# EUROPEAN PATENT SPECIFICATION

(11) **EP 1 870 106 B1**
(45) Date of publication and mention of the grant of the patent: **20.05.2009**
(21) Application number: 06705745.5
(22) Date of filing: 24.02.2006
(51) Int. Cl.: A61K 38/18, A61K 38/08, A61K 38/25, A61P 25/28

(54) **COMBINATION OF EGF/GHRP-6 FOR NEUROREGENERATION OF CENTRAL NERVOUS SYSTEM**
KOMBINATION VON EGF/GHRP-6 FÜR DIE NEUROREGENERATION DES ZENTRALNERVENSYSTEMS
COMBINAISON D'EGF/GHRP-6 POUR LA NEUROREGENERATION DU SYSTEME NERVEUX CENTRAL

(30) Priority: 02.03.2005 CU 432005
(43) Date of publication of application: 26.12.2007
(73) Proprietor: CENTRO DE INGENIERIA GENETICA Y BIOTECNOLOGIA (CIGB), Ciudad de la Habana 10600 (CU)
(72) Inventor: GARCÍA DEL BARCO HERRERA, Diana, Cuidad De La Habana 11 600 (CU); GUILLEN NIETO, Gerardo Enrique, Ciudad De La Habana 10 400 (CU); BERLANGA ACOSTA, Jorge A. Avenida 31, Ciudad De La Ha Habana 11600 (CU); FREYRE ALMEIDA, Freya De Los Milagros, Playa Ciudad De La Habana 11600 (CU); CIBRIAN VERA, Danay Avenida 15 entre 82y90 Edif., Ciudad De La Habana 12800 (CU); PENTÓN ARIAS, Eduardo Avenida 31, Ciudad De La Habana 11600 (CU)
(74) Representative: Hatzmann, Martin
(86) International application number: PCT/CU2006/000001
(87) International publication number: WO 2006/092106

(56) References cited:
- WO-A-00/30675
- FRAGO LAURA M ET AL: "Growth hormone (GH) and GH-releasing peptide-6 increase brain insulin-like growth factor-I expression and activate intracellular signaling pathways involved in neuroprotection" ENDOCRINOLOGY, vol. 143, no. 10, October 2002 (2002-10), pages 4113-4122, XP002395581 ISSN: 0013-7227 cited in the application
- HARVEY STEVE ET AL: "Neural growth hormone: an update." JOURNAL OF MOLECULAR NEUROSCIENCE : MN. FEB 2003, vol. 20, no. 1, February 2003 (2003-02), pages 1-14, XP009071227 ISSN: 0895-8696
- SCHNEIDER HARALD JOERN ET AL: "Central effects of the somatotropic system." EUROPEAN JOURNAL OF ENDOCRINOLOGY, vol. 149, no. 5, November 2003 (2003-11), pages 377-392, XP002395582 ISSN: 0804-4643
- PLATA-SALAMAN C R: "EPIDERMAL GROWTH FACTOR AND THE NERVOUS SYSTEM" PEPTIDES, ELSEVIER, AMSTERDAM, US, vol. 12, no. 3, May 1991 (1991-05), pages 653-663, XP001181067 ISSN: 0196-9781 cited in the application
- KNAPP PAMELA E ET AL: "Epidermal growth factor promotes oligodendrocyte process formation and regrowth after injury." EXPERIMENTAL CELL RESEARCH. 10 JUN 2004, vol. 296, no. 2, 10 June 2004 (2004-06-10), pages 135-144, XP002395583 ISSN: 0014-4827 cited in the application
- RAINETEAU OLIVIER ET AL: "Neurogenesis in hippocampal slice cultures" MOLECULAR AND CELLULAR NEUROSCIENCE, vol. 26, no. 2, June 2004 (2004-06), pages 241-250, XP002395584 ISSN: 1044-7431 cited in the application
- KUHN H G ET AL: "Epidermal growth factor and fibroblast growth factor-2 have different effects on neural progenitors in the adult rat brain" JOURNAL OF NEUROSCIENCE, NEW YORK, NY, US, vol. 17, no. 15, 1997, pages 5820-5829, XP002229396 ISSN: 0270-6474
- ARSENIJEVIC Y AND WEISS S: "Insulin-like growth factor-I is a differentiation factor for postmitotic CNS stem cell-derived neural precursors: Distinct actions from those of brain-derived neurotrophic factor" JOURNAL OF NEUROSCIENCE, NEW YORK, NY, US, vol. 18, no. 6, 15 March 1998 (1998-03-15), pages 2118-2128, XP002146610 ISSN: 0270-6474
- ROM POULSEN F ET AL: "Glutamate receptor antagonists and growth factors modulate dentate granule cell neurogenesis in organotypic, rat hippocampal slice cultures" BRAIN RESEARCH, AMSTERDAM, NL, vol. 1051, no. 1-2, 27 July 2005 (2005-07-27), pages 35-49, XP004984668 ISSN: 0006-8993

## Description

### Field of the invention

The present invention relates to medicine, and more specifically to neurology and is directed to stimulate central nervous system neuregeneration after autoimmune damage, particularly for the treatment and prevention of relapses in patients with multiple sclerosis and optic neuromyelitis by administering compositions containing Epidermal Growth Factor and Growth Hormone Releasing Peptide-6.

### Background art

Multiple Sclerosis (MS) and Optic Neuromyelitis (NO) are autoimmune demyelinating diseases that affect young people, fundamentally women, resulting in incapacitation and invalidities that progress in time. MS incidence strongly correlates with advanced parameters of industrialization and development in first world countries.

The Central Nervous System (CNS) is a privileged immunological site where autoimmune reactions are infrequently found. This occurs when by undetermined causes, cellular and humoral regulatory mechanism fail, which determine that auto-reactive cells against myelin antigens that are peripherically generated (which is often the case), cross through the Blood Brain Barrier (BBB) in the form of activated lymphocytes and find a target in the central nervous system parenchyma. Thereafter a series of cascade like events occurs which result in demyelination, reactive astrocytosis, and cell death of neurons and oligodendrocytes.

The auto-immune reaction within the central nervous system is directed against myelin antigens so that in first instance the damage is limited to myelin sheets which cover the axons of the main neurons, to the oligodendrocyte which is responsible for myelin production and to other group of neurons that become injured in an unspecific manner due to the expansion of the autoimmune reaction.

The ensuing demyelination and the neuronal death either by necrosis and/or apoptosis result in loss of sense and/or motor function that randomly affect specific regions of the human body.

The remyelination process in MS and ON is in general limited and transient. This remyelination process, although possible, depends on the balance between auto-reactive astrocytes and the oligodendrocytes (John G.R., Shankar S.L., Shafit-Zagardo B., Massimi A., Lee S.C., Raine C.S. et al. (2002) Multiple sclerosis: re-expression of a developmental pathway that restricts oligodendrocyte maturation Nature Medicine 8(10):1115-1121).

Among the neural regeneration strategies, the treatment with growth factors such as Epidermal Growth Factor (EGF) and bovine-Fibroblast Growth Factor (bFGF) have been very promising proposals demonstrating that multi-potent, undifferentiated cell lineages isolated from the brain cortex are responsive to these growth factors differentiating toward different cell lineages such as type I and II astrocytes, myelinating oligodendrocytes, and different neuronal types (Mehler M.F., Gokhan S. (1999) Postnatal cerebral cortical multipotent progenitors: regulatory mechanisms and potential role in the development of novel neural regenerative strategies. Brain Pathol; 9(3):515-526).

Growth Hormone Releasing Peptide-6 (GHRP-6) increases the expression of Insulin-like Growth Factor 1 (IGF-1) in the central nervous system (Frago L.M., Paneda C., Dickson S.L., Hewson A.K., Argente J., Chowen J.A. (2002) Growth hormone (GH) and GH-releasing peptide-6 increase brain insulin-like growth factor-I expression and activate intracellular signaling pathways involved in neuroprotection. Endocrinology 143(10):4113-4122).

IGF-1 is involved in processes like oligodendrocytes maturation (Wilson H.C., Onischke C., Raine C.S. (2003) Human oligodendrocyte precursor cells in vitro: phenotypic analysis and differential response to growth factors. Glia 44(2):153-165), blocking of Tumor Necrosis Factor (TNF-α) dependent apoptosis pathways thus protecting the MS and ON from damage induced by TNF-α (Ye P, D'Ercole A.J. (1999) Insulin-like growth factor 1 protects oligodendrocytes from tumor necrosis factor-alpha-induced injury. Endocrinology 140(7):3063-3072), and further reduces the expression of molecules of the major histocompatibility complex class I (MHC-I) (Ito T, Ito N, Bettermann A, Tokura Y, Takigawa M, Paus R. (2004) Collapse and restoration of MHC class-I-dependent immune privilege: exploiting the human hair follicle as a model. Am. J. Pathol 164(2):623-634). In experimental models of autoimmune encephalitis (Experimental Autoimmune Encephalitis or EAE) IGF-1 reduces the vascular endothelium lesion of the BBB, the number and the size of sclerosis plaques, pathognomonic lesions of Multiple Sclerosis. (Li W, Quigley L, Yao D.L, Hudson L.D, Brenner M, Zhang B.J et al. (1998) Chronic relapsing experimental autoimmune encephalonyelitis: effects of insulin-like growth factor-1 treatment on clinical deficits, lesion severity, glial responses, and blood brain barrier defects. J Neuropathol Exp Neurol 57(5):426-438).

When systemically administered, GHRP-6 increases levels of endogenous adrenocorticotrophin hormone (ACTH) (Martins M.R, Pinto A.C, Brunner E, Silva M.R, Lengyel A.M. (2003) GH-releasing peptide (GHRP-6)-induced ACTH release in patients with addison's disease: effect of glucocorticoid withdrawal. J Endocrinol Invest. 26(2):143-147). ACTH as an endogenous steroid releasing factor has a beneficial effect for counteracting the auto-reactive disorders and for a long time has been the traditional therapy for MS (Oishi C, Sakuta M. (2003) Steroid therapy for multiple sclerosis. Nippon Rinsho 61(8):1361-1366).

EGF is locally synthesized in the central nervous system (CNS) by microglias, blood-derived macrophages and also by some neurons. As it passes through the BBB and the ventricle lying membranes, EGF is able to flow the CNS. EGF has been attributed a certain number of physiological functions as CNS development, maintenance and differentiation of CNS parenchymal cells, actions which are very much related to neural regeneration processes and to survival mechanisms triggered upon insults (Plata-Salaman C.R. (1991) Epidermal growth factor and the nervous system. Peptides 12(3):653-663).

EGF stimulates cell proliferation and survival within the CNS (Thorne R.G, Hrabetova S, Nicholson C. (2004) Diffusion of Epidermal Growth Factor in Rat Brain Extracellular Space Measured by Integrative Optical Imaging. J Neurophysiol 92(6):3471-3481).

EGF-stimulated oligodendrocytes gain an enhanced remyelinating potential. EGF contribute to oligodendrocyte proliferation process so facilitating the beginning of cellular division and further differentiation into specialized cells as mature oligodendrocytes, astrocytes and Schwann cells. EGF promotes events such as neurogenesis given by the generation of novel neurons (Crang A.J., Gilson J.M., Li W.W., Blakemore W.F. (2004) The remyelinating potential and in vitro differentiation of MOG-expressing oligodendrocyte precursors isolated from the adult rat CNS. Eur J Neurosci 20(6):1445-1460; Raineteau O., Rietschin L., Gradwohl G., Guillemot F., Gahwiler B.H. (2004) Neurogenesis in hippocampal slice cultures. Mol Cell Neurosci 26(2):241-250). These events are likely more seen after the oligodendrocytes have experienced damage, suggesting that the EGF-mediated effects on the regenerative process are consequent to interactions between the EGF and a signal transduction system which is specifically activated in injured oligodendrocytes. This also suggests that the modulation of this signal transduction system may amplify mechanisms toward remyelination (Wang K., Wang J..J, Wang Y., He Q.H., Wang X., Wang X.M. (2004) Infusion of epidermal growth factor and basic fibroblast growth factor into the striatum of parkinsonian rats leads to in vitro proliferation and differentiation of adult neural progenitor cells. Neurosci Lett 364(3):154-158; Knapp P.E., Adams M.H. (2004) Epidermal growth factor promotes oligodendrocyte process formation and regrowth after injury. Exp Cell Res 296(2):135-144).

During the last few years complex therapeutic interventions have been proposed as combination therapies and/or system therapies that are far from redundant and strengthen the therapeutical approach, thus allowing the access to complex pathophysiological problems at the nodes or key points related to the disease of reference. A combined therapy with various growth factors or the combination of one of them with alternative molecules having positive trophism for the CNS is lacking as yet.

In all cases, an ideal therapy would reduce the symptoms associated with the initial outbreak and would reduce at a minimum the relapses frequency. Therefore, there is a need for developing a more efficient method for used in the treatment and for preventing recurrence of different clinical forms of Multiple Sclerosis and Optic Neuromyelitis.

The administration of the combination comprising therapeutically effective concentrations of EGF and the secretagogue GHRP-6 has been previously suggested for the prophylaxis and the treatment of tissue damages due to arterial blood supply deficit (WO 02/053167).

### Summary of the invention

The present invention is based on a method in which the co-administration of EGF and GHRP-6 represent and improved treatment for auto-immune disorders of the CNS. This combination protects and reverts the auto-immune associated damages in chronic processes of the CNS, particularly in multiple sclerosis and optic neuromyelitis.

As compared to each ingredient alone, the combination produces a more long-lasting efficacy and a substantial reduction of relapses - in other words, it triggers regenerative events in a more efficient way. As it is used herein, the term "more long lasting efficacy", means that the active ingredients lead to the amelioration of the MS and ON associated symptoms during a longer period of time, even conferring protection to avoid relapses episodes. This will ensure the restoration of the affected neurological functions as a consequence of demyelination and neuronal losses by apoptosis / necrosis brought about by the auto-immune damage. On the other hand, the active ingredients of the pharmaceutical combination are autologous proteins and peptides endowed with natural regulatory cells stimulatory capabilities so they would be stimulated to proliferate after the exogenous peptide administration. By this way, the auto-immune response may be counteracted as the regulatory T cells role is to constitutively mediate the immunological tolerance (Jörn G., Benedikt B., Bruno K. (2004) Medullary Epithelial Cells of the Human Thymus Express a Highly Diverse Selection of Tissue-specific Genes Colocalized in Chromosomal Clusters. J Exp Med 199(2):155-166.); (Dayne M., Christophe B. (2004) Back to Central Tolerance. Immunity 20:509-516); (Mark S.A., Emily S.V., Ludger K., Zhibin C., Stuart P.B., Shannon J.T. et al. (2002) Projection of an Immunological Self Shadow Within the Thymus by the Aire Protein. Science 298:1395-1400) (Shimon S. (2004) Naturally arising CD4+ regulatory T cells for immunologic self-tolerance and negative control of immune responses. Annual Review of Immunology 22:531-562)*.*

Due to the synergic effect between EGF and GHRP-6 in relation to neuro-trophic/ neuro-regenerative events, this combination is useful in accelerating neurogenesis processes, which assists in the regaining functions of motor and sensory nerves lost by the auto-immune generated damage or lesion. The combination of EGF and GHRP-6 could be associated with any anti-oxidant therapy.

The therapeutic administration of the combination toward neuro-regeneration and neuro-protection requires of repeated administration schedules. As described in the present invention, the active ingredient referred to as EGF may be derived from any animal species, including ovine, bovine, porcine and human, in its native sequence or its variants and from any source such as synthetic, natural or recombinant. The preferred form in this case is the human EGF in its native sequence, and most of all human recombinant EGF. The active ingredient referred to as the growth hormone releasing peptide (GHRP) is the hexapeptide having the following sequence: His-D-Trp-Ala-Trp-D-Phe-Lys-NH₂, obtained through chemical synthesis.

In a particular setting, the therapeutic doses administered during the MS and ON crises are in the range of 5-200µg/kg/day for EGF and between 0.5-350 µg/kg/day for the GHRP-6 for 20-30 days.

In another setting of this invention, the doses administered within the inter-crises stages in order to prevent relapses in multiple sclerosis are in the range between 0.5-50µg/kg/day for each of the ingredients for a period of up to 130 days. The combination must be administered as bolus. The administration routes will be parenteral, in peripheral veins, intramuscular or intraperitoneal. The vehicles involved in the administration include normal saline solution, Lactate Ringer solution, human plasma, human albumin solution, dextrose 5%, gelatin solution or the mixtures thereof.

With the expectation to achieve the highest therapeutic efficacy for multiple sclerosis, in relapsing-remitting or secondary progressive clinical forms, the first administration must be done as coincident with the prodroms of the disease (personalized). In the remission phases, sustained therapeutic schemes are proposed with the above mentioned doses as preventive of the relapses.

In other multiple sclerosis clinical forms sustained treatment schemes are proposed involving therapeutic doses.

In line with other setting of the present invention, the combination EGF/GHRP-6 induces the proliferation or natural and adaptive regulatory T cells that prevent the onset of EAE severe clinical forms in adoptive transfer experiments.

The combination EGF/GHRP-6 can be used within a single pharmaceutical composition or by mixing the independent ingredients just before use. The active ingredients combination can be used by mean of slow releasing devices. If the formulation is freeze-thawed, this must be diluted just before use.

### Detailed description of particular embodiments / Examples.

### Example 1. Therapeutical efect of EGF/GHRP-6 pharmaceutical combination in an Experimental Autoimmune Encephalytis (EAE) biomodel.

In order to asses therapeutical efficiency of the EGF/GHRP-6 pharmaceutical combination an animal model of EAE was set up which represents the animal counterpart of the multiple sclerosis human disease.

Female Lewis Rats (130g), were subcutaneously immunized with guinea pig spinal cord homogenate (5mg) in PBS (50%) and complete Freund adjuvant (50%), during days 0 and 6. Ten days after the first immunization the therapeutical scheme was initiated using the combination EGF/GHRP-6 (200µg/kg/day-740µg/kg/day), the independent active ingredients EGF (200µg/kg/day), GHRP-6 (740µg/kg/day) and placebo (PBS). This therapeutical scheme was followed for 10 days, using intraperitoneal administration. Clinical scores was based on the following grading: 0; no symptom, 1; tail paralysis, 2; paralysis of any of the hind limbs, 3; full paralysis of the hind limbs, 4; complete paralysis of the fore and hind limbs 5; moribund or death. Weight loss and vesical and rectal sphincter incontinency, which are also clinical signs of the disease, are scored by adding 0.5 to the clinical index previously described. Forty days after the first immunization the animals were anesthetized and euthanized, the encephalon and spinal cord were processed for histopathological study (10% de formalin, H & E y Luxol Blue staining). For the histopathological analysis the following parameters were consider: number and size of perivascular inflammatory infiltrates, number of demyelination lesions, number of apoptotic neurons and glial cells and astrocytes reactivity. The microscopic study was blindly conducted.

As showed in table 1 the combination EGF/GHRP-6 protects the animals experimentally induced to develop EAE, only 50% of these animals develops the slightest form of the disease, while the rest remains unaltered. By the contrary in the rest of groups the disease has a 100% of incidence (groups treated with the independent active ingredients and placebo). Mean clinical index in the combination treated animals EGF/GHRP-6 was 0.37 ± 0.47. For the independent ingredients treated Groups the mean clinical index were 1.37 ± 1.7 for the EGF and 1.5 ± 1.6 for GHRP-6 and placebo treated group showed mean clinical index 1.7 ± 1.4. Eight rats were allocated for each group. The statistical comparison between the groups was p<0.001. The Newman Keuls multiple comparison test was used.

**Table 1. Clinical summary of the therapeutical effect of rats induced to develop EAE.**

| Groups | Incidence (%) | Days of Debut (Mean ± SD) | Clinical Scores | | | |
|---|---|---|---|---|---|---|
| | | | Mean ± SD | maximum | minimum | Disease duration |
| Control | 0 | 0 | 0 | 0 | 0 | 0 |
| EGF | 100 | 12.5 ± 0.57 | 1.37 ± 1.7 | 5 | 1 | 12.7 ± 4.1 |
| GHRP-6 | 100 | 13.7 ± 2.3 | 1.5 ± 1.6 | 4 | 0.5 | 15.5 ± 6.7 |
| EGF/GHRP-6 | 50 | 12 ± 0 | 0.37 ± 0.47 | 1 | 0 | 9 ± 1.4 |
| Placebo | 100 | 12.2 ± 0.5 | 1.7 ± 1.4 | 4 | 0.5 | 13.2 ± 8.1 |

As shown in table 2 the results of the pathological study analysis of the encephalon and spinal cord of the animals included in the different groups show that even existing the same situation regarding to reactive astrocytes, the number (p=0.028 unpaired T test) and size of vascular cuff in EGF/GHRP-6 treated animals are smaller than the placebo treated group

**Table 2. Perivascular inflammatory infiltrates in the brain and spinal cord of the animals.**

| Groups | N° of perivascular inflammatory infiltrates |
|---|---|
| | (Means ± SD) |
| Control | 0 |
| EGF | 4.5 ± 1.9 |
| GHRP-6 | 2.25 ± 1.25 |
| EGF/GHRP-6 | 2 ± 0.8 |
| Placebo | 5 ± 2.1 |

This experiment demonstrated that the EGF/GHRP pharmaceutical combination protects animals from developing severe clinical forms of the disease. The mechanisms underlying this protective effect are the increased production of myelin by oligodendrocytes and the subsequent remyelination of the affected nervous structures. Another mechanism is related to the integrity of BBB, to avoid the passage of auto-reactive cells to the brain parenchyma.

### Example 2. Protective effect of the EGF/GHRP-6 pharmaceutical combination in a EAE animal model prophylactic scheme.

In order to asses the prophylactic effect of the EGF/GHRP-6 combination in an EAE animal model representing the MS human disease, female Lewis Rats (130g) were subcutaneously immunized with guinea pig spinal cord homogenates (5mg) in PBS (50%) and a complete Freund adjuvant (50%), on days 0 and 6. Ten days before the first immunization the prophylactic scheme was initiated using the EGF/GHRP-6 combination (200µg/kg/day-740µg/kg/day) and the separate active ingredients EGF (200µg/kg/day), GHRP-6 (740µg/kg/day) and placebo (PBS). This prophylactic scheme was followed for 10 days (-10 to -1 day before the first immunization), using the intraperitoneal administration route. Clinical scores was based on the following grading: 0; no symptom, 1; tail paralysis , 2; paralysis of any of the hind limbs, 3; full paralysis of the hind limbs, 4; complete paralysis of the fore and hind limbs 5; moribund or death. Weight loss and vesical and rectal sphincter incontinency, which are also clinical signs of the disease, are scored by adding 0.5 to the clinical index previously described. Forty days after the first immunization the animals were anesthetized and euthanized, the encephalon and spinal cord were processed for histopathological study (10% de formalin, H & E y Luxol Blue staining). For the histopathological analysis the following parameters were consider: number and size of perivascular inflammatory infiltrates, number of demyelination lesions, number of apoptotic neurons and glial cells and astrocytes reactivity. The microscopic study was blindly conducted.

As shown in table 3 the EGF/GHRP-6 pharmaceutical combination used in the prophylactic scheme protected the animals induced to develop EAE. One-hundred percent of EGF/GHRP-6 treated animals developed a mild form of the disease (0.5-1 clinical scores) In contrast, for the groups treated with the single ingredients, 75% developed a severe clinical form of EAE (3-4 clinical scores). The mean clinical index found for the animals treated with the pharmaceutical combination EGF/GHRP-6 was 0.68 ± 0.25. For the single ingredient treated groups the mean clinical indices were 2.8 ± 0.99 for the EGF and 2.7 ± 1.03 for GHRP-6 (P=0.0003 compared to EGF/GHRP-6 treated animals) and the placebo treated group showed a mean clinical index of 3 ± 1.4 (p =0.0011 compared to EGF/GHRP-6 treated animals). Eight rats were used for each group. For the statistical comparison the Mann Whitney T test was used, comparing the group treated with the combination and the placebo group a value of p=0.0011 was obtained and for the groups treated with the independent active ingredients p = 0.0003 values were obtained as compared to the group treated with the pharmaceutical combination

**Table 3. Clinical summary of the prophylactic effect in rats induced to develop EAE.**

| Groups | Incidence (%) | Days of Debut (Mean ± SD) | Clinical scores | | |
|---|---|---|---|---|---|
| | | | Means ± SD | maximum | minimum |
| Control | 0 | 0 | 0 | 0 | 0 |
| EGF | 100 | 11.5 ± 0.57 | 2.8 ± 0.99 | 4 | 1 |
| GHRP-6 | 100 | 11.7 ± 2.3 | 2.7 ± 1.03 | 4 | 1 |
| EGF/GHRP-6 | 100 | 10 ± 0 | 0.68 ± 0.75 | 1 | 0.5 |
| Placebo | 100 | 12.2 ± 0.5 | 3 ± 1.4 | 5 | 1 |

As observed in table 4 the pathological analysis of the encephalon and spinal cord in the experimental groups show that even in the same case of reactive astrocytes, the number (p=0.025 unpaired T test) and size of the perivascular cuff in EGF/GHRP-6 prophylactically treated animals are fewer and smaller than the placebo treated group.

**Table 4. Perivascular inflammatory infiltrates in the brain and the spinal cord of prophylactically treated animals.**

| Groups | N° of perivascular inflammatory infiltrates |
|---|---|
| | (Means ± SD) |
| Control | 0 |
| EGF | 4 ± 16 |
| GHRP-6 | 2.7 ± 1.95 |
| EGF/GHRP-6 | 1.5 ± 1 |
| Placebo | 5 ± 2.1 |

This experiment demonstrated that the EGF/GHRP-6 pharmaceutical combination used prophylactically protects animals from developing EAE in its most severe clinical form. Moreover, it demonstrated a strong correlation between the clinical evolution and histological findings. The mechanisms explaining this protective effect are related with the induction of differentiation of neuronal precursor cells toward oligodendrocytes which will be preconditioned and active in myelin production. The conservation of the integrity of the BBB will prevent the passage of autoreactive cells towards the brain parenchyma this is another event explaining the protective rol of the EGF/GHRP-6 pharmaceutical combination used preventively.

### Example 3. Dose study, synergism - potentiation between the active principles of the pharmaceutical combination

Looking for a range of doses for the pharmaceutical combination that would be efficient for the therapeutic effects, it was used in the afore mentioned EAE model Female Lewis Rats (130g), were subcutaneously immunized with guinea pig spinal cord homogenate (5mg) in PBS (50%) and complete Freund adjuvant (50%), during days 0 and 6. Ten days after the first immunization the therapeutical scheme was initiated using the combination EGF/GHRP-6 combination in different concentrations.
EGF/GHRP-6 (400 µg/kg/day-1480 µg/kg/day).
EGF/GHRP-6 (200 µg/kg/day-740 µg/kg/day).
EGF/GHRP-6 (100 µg/kg/day-340 µg/kg/day)
EGF/GHRP-6 (50 µg/kg/day-170 µg/kg/day)
EGF/GHRP-6 (25 µg/kg/day-85 µg/kg/day)
EGF/GHRP-6 (12 µg/kg/day-40 µg/kglday)

This therapeutical scheme was followed for 10 days, using intraperitoneal administration. Clinical scores was based on the following grading: 0; no symptom, 1; tail paralysis, 2; paralysis of any of the hind limbs, 3; full paralysis of the hind limbs, 4; complete paralysis of the fore and hind limbs 5; moribund or death. Weight loss and vesical and rectal sphincter incontinency, which are also clinical signs of the disease, are scored by adding 0.5 to the clinical index previously described. Forty days after the first immunization the animals were anesthetized and euthanized, the encephalon and spinal cord were processed for histopathological study (10% de formalin, H & E y Luxol Blue staining). For the histopathological analysis the following parameters were consider: number and size of perivascular inflammatory infiltrates, number of demyelination lesions, number of apoptotic neurons and glial cells and astrocytes reactivity. The microscopic study was blindly conducted.

In the EGF/GHRP-6 (400 µg/kg/day-1480 µg/kg/day) treated group, 25% of the animals remained unaltered, 75% of the animals showed a mild form of the disease (0.5-1). The mean clinical index was 0.62 ± 0.44. In the EGF/GHRP-6 (200 µg/kg/day-740 µg/kg/day) treated group, 25% of the animals remained unaltered, 75% of the animals showed the mild form of the disease (0.5-1). The mean clinical index was 0.5 ± 0.37.

In the EGF/GHRP-6 (100 µg/kg/day-340 µg/kg/day) treated group, 12.5% of the animals remained unaltered, 87.5% of the animals showed the mild form of the disease (0.5-1). The mean clinical index was 0.62 ± 0.35.

In the EGF/GHRP-6 (50 µg/kg/day-170 µg/kg/day) treated group, 100% of the animals developed EAE, 12.5% with a intermediate clinical form (2) and the rest showed the mild form of the disease (0.5-1). The mean clinical index was 0.93 ± 0.49.

In the EGF/GHRP-6 (25 µg/kg/day-6 85 µg/kg/day) treated group, 100% of the animals developed EAE, 37.5% with a intermediate clinical form (2) and the rest showed the mild form of the disease (0.5-1). Mean clinical index was 1.25 ± 0.65.

In the EGF/GHRP-6 (12 µg/kg/day-40 µg/kg/day) treated group, 100 % of the animals developed EAE, 12.5% with the most severe clinical form (3), 37.5% with an intermediate clinical form (2) and the rest showed the mild form of the disease (0.5-1). The mean clinical index was 1.37 ± 0.87.

Tables 5 and 6 show the clinical and the histopathological results. The histopathological analysis showed that there were no statistical differences in the number of the lymphocytic perivascular infiltrates in EAE-induced and treated groups with EGF/GHRP-6 (400 µg/kg-1480 µg/kg, 200 µg/kg-740 µg/kg, 100 µg/kg-340 µg/kg, 50 µg/kg-170 µg/kg y 25 µg/kg-85 µg/kg). In the case of the group treated with the EGF/GHRP-6 pharmaceutical combination (12 µg/kg-40 µg/kg), the number of perivascular infiltrates showed a trend to be higher (p=0.040), but this difference was not statistically significant. These results demonstrate that there is a dose range of 50-400 µg/kg/day for the EGF and of 170 µg/kg/day-1.4 mg/kg/day for the GHRP-6, which enables the formulation of the combination that maintain its usefulness for the protection against induction of EAE (Table 5).

**Table 5. Clinical summary of the dose response and synergism - potentiation between the separate ingredients of the EGF/GHRP-6 combination**

| Groups EGF/GHRP-6 | Incidence | Clinical score | | |
|---|---|---|---|---|
| | (%) | Mean ±SD | maximum | minimum |
| EGF/GHRP-6 (400µg/kg-1480µg/kg) | 75 | 0.62 ± 0.44 | 0 | 1 |
| EGF/GHRP-6 (200µg/kg-740µg/kg) | 75 | 0.5 ± 0.37 | 0 | 1 |
| EGF/GHRP-6 (100µg/kg-340µg/kg) | 87.5 | 0.65 ± 0.35 | 0 | 1 |
| EGF/GHRP-6 (50µg/kg-170µg/kg) | 100 | 0.93 ± 0.49 | 0.5 | 2 |
| EGF/GHRP-6 (25µg/kg-85µg/kg) | 100 | 1.25 ± 0.65 | 0.5 | 2 |
| EGF/GHRP-6 (12µg/kg-40 µg/kg) | 100 | 1.3 ± 0.87 | 0.5 | 3 |

**Table 6. Perivascular inflammatory infiltrates in the brain and the spinal cord of each experimental group.**

| Groups EGF/GHRP-6 | |
|---|---|
| | N° of perivascular inflammatory infiltrates |
| | (Means ± SD) |
| EGF/GHRP-6 (400µg/kg-1480µg/kg) | 2.7 ± 0.95 |
| EGF/GHRP-6 (200µg/kg-740µg/kg) | 2.7 ± 0.5 |
| EGF/GHRP-6 (100µg/kg-340µg/kg) | 2.2 ± 1.2 |
| EGF/GHRP-6 (50µg/kg-170µg/kg) | 3 ± 1.4 |
| EGF/GHRP-6 (25µg/kg-85µg/kg) | 3.2 ± 0.95 |
| EGF/GHRP-6 (12µg/kg-40 µg/kg) | 4 ± 0.8 |

### Example 4. Evaluation of the effect of the EGF-GHRP-6 pharmaceutical combination in generating a natural regulatory T cell response.

Twenty female Lewis Rats (130g), were subcutaneously immunized with guinea pig spinal cord homogenates (5mg) in PBS (50%) and the complete Freund adjuvant (50%), on days 0 and 6. Ten days after the first immunization, the therapeutic scheme was started using the EGF/GHRP-6 (200µg/kg/day-740µg/kg/day) combination and followed for other 10 days by its intraperitoneal administration in ten of the immunized rats (Group A). The other 10 rats were PBS treated as placebo (Group B). A week after the last administration the animals from both groups were anesthetized for bleeding and to thereby obtain peripheral blood mononuclear lymphocytes. The lymphocytes derived from groups A and B were treated for the segregate the CD4⁺ cells.

The analysis by FACS of the CD4⁺CD25⁺ cells was 14.67% in group A and 3,8% in the PBS treated group (group B).

Other Female Lewis Rats (130g), were subcutaneously immunized with guinea pig spinal cord homogenates (5mg) in PBS (50%) and the complete Freund adjuvant (50%), on days 0 and 6. Ten days after the first immunization a sub-group (n=8) was adoptively transferred intravenously with 500 000 CD4⁺ cells from group A. Another sub-group (n=8) was adoptively transferred intravenously with 500 000 CD4⁺ cells from group B.

Clinical scores was based on the following grading: 0; no symptom, 1; tail paralysis, 2; paralysis of any of the hind limbs, 3; full paralysis of the hind limbs, 4; complete paralysis of the fore and hind limbs 5; moribund or death. Weight loss and vesical and rectal sphincter incontinency, which are also clinical signs of the disease, are scored by adding 0.5 to the clinical index previously described. Forty days after the first immunization the animals were anesthetized and euthanized, the encephalon and spinal cord were processed for histopathological study (10% de formalin, H & E y Luxol Blue staining). For the histopathological analysis the following parameters were consider: number and size of perivascular inflammatory infiltrates, number of demyelination lesions, number of apoptotic neurons and glial cells and astrocytes reactivity. The microscopic study was blindly conducted.

As shown in table 7, the transference of CD4⁺cells in EAE induced animals and treated with the pharmaceutical combination, protects the host from developing EAE. Only 50% of the animals transferred with CD4+ cells derived from group A developed a mild clinical form of EAE (0.5-1 clinical score), the resting 50% remained unaltered. In contrast 100% of the animals adoptively transferred with CD4⁺ derived from group B, developed EAE, 62.5% with the severe clinical form (2-4 clinical score) and 37.5% with a mild clinical form (0.5-1 clinical score). The mean clinical index was 0.31 ± 0.34 in the sub-group adoptively transferred with CD4⁺ derived from group A. The mean clinical index was 2.1 ± 0.99 in the sub-group adoptively transferred with CD4⁺ derived from group B (P =0.0003). Mann Whitney T test was used for the statistical analysis.

**Table 7. Protective effect of the adoptive cell transfer.**

| Groups | Incidence (%) | Days of Debut (Mean ± SD) | Clinical score | | |
|---|---|---|---|---|---|
| | | | Mean ± SD | maximum | minimum |
| Group A | 50 | 10 ± 0 | 0.31 ± 0.34 | 1 | 0 |
| Group B | 100 | 11.5 ± 0.57 | 2.1 ± 0.99 | 4 | 1 |

As shown in table 8, the histological analysis of the encephalon and spinal cord in the experimental groups showed that even existing the same situation regarding reactive astrocytes, the number (p=0.0001) and size of the vascular cuffs in the host rats for the CD4⁺ derived from group A are smaller than in rats that are adoptively transferred with CD4⁺ cells derived from group B

**Table 8. Perivascular inflammatory infiltrates in the brain and the spinal cord of each experimental group.**

| Groups | N° of perivascular inflammatory infiltrates |
|---|---|
| | (Means ± SD) |
| group A CD4⁺ host | 2.1 ± 0.8 |
| group B CD4⁺ host | 6.5 ± 1.7 |

These results demonstrated that the treatment with the pharmaceutical combination is able to induce proliferation of natural regulatory T cells, which protect from developing severe clinical forms of EAE in adoptive transfer experiments.

## Claims

1. Use of Epidermal Growth Factor (EGF) and Growth Hormone Releasing Peptide-6 (GHRP-6) for the manufacture of a medicament for the treatment and amelioration of Central Nervous System disorders in a patient suffering from a symptom or complications related to demyelination, neuronal degeneration and neuronal cell death by apoptosis or necrosis wherein the aforementioned diseases have an autoimmune etiology.

2. Use according to claim 1, wherein the EGF is human EGF.

3. Use according to claim 2, wherein the human EGF is obtained from a natural source, by recombinant technology or chemical synthesis.

4. Use according to any one of claims 1-3, wherein the central nervous system disorder is:
a. Multiple Sclerosis;
b. Optic Neuromyelitis.

5. Use according to any one of claims 1-4, wherein the combination EGF-2GHRP-6 is administered intravenously, intramuscularly or intraperitoneally or by using device for controlled release.

6. Use according to any one of claims 1-5, wherein the medicament is administered parenterally in a therapeutic scheme for a period of 20 to 30 days, in a dose range of between 5-10 µg of each independent ingredient, per kilogram of patient body weight per day.

7. Use according to any one of claims 1-6, wherein the medicament is parenterally administered during remissions, for a period of up to 130 days, in a dose range of between 1-5 µg of each independent ingredient, per kilogram of patient body weight per day.

8. Use according to claim 1, wherein the medicament induces proliferation of the natural and adaptive regulatory T cells.

## Patentansprüche

1. Verwendung von epidermalem Wachstumsfaktor (EGF) und Wachstumshormon freisetzendem Peptid-6 (GHRP-6) für die Herstellung eines Medikaments für die Behandlung und Besserung von Störungen des Zentralnervensystems bei einem Patienten, der an einem Symptom oder Komplikationen leidet, die mit Demyelinierung, neuronaler Degeneration und neuronalem Zelltod durch Apoptose oder Nekrose in Zusammenhang stehen, wobei die vorstehend erwähnten Erkrankungen eine autoimmune Ätiologie aufweisen.

2. Verwendung nach Anspruch 1, wobei das EGF humanes EGF ist.

3. Verwendung nach Anspruch 2, wobei das humane EGF aus einer natürlichen Quelle, durch rekombinante Technologie oder chemische Synthese erhalten wird.

4. Verwendung nach einem der Ansprüche 1-3, wobei es sich bei der Störung des Zentralnervensystems um:
a. Multiple Sklerose;
b. Neuromyelitis optica
handelt.

5. Verwendung nach einem der Ansprüche 1-4, wobei die Kombination EGF-GHRP-6 intravenös, intramuskulär oder intraperitoneal oder unter Verwendung einer Vorrichtung für eine kontrollierte Freigabe verabreicht wird.

6. Verwendung nach einem der Ansprüche 1-5, wobei das Medikament in einem therapeutischen Schema für einen Zeitraum von 20 bis 30 Tagen in einem Dosisbereich zwischen 5-10 µg von jedem unabhängigen Inhaltsstoff pro Kilogramm Körpergewicht des Patienten pro Tag parenteral verabreicht wird.

7. Verwendung nach einem der Ansprüche 1-6, wobei das Medikament während Remissionen für einen Zeitraum von bis zu 130 Tagen in einem Dosisbereich zwischen 1-5 µg von jedem unabhängigen Inhaltsstoff pro Kilogramm Körpergewicht des Patienten pro Tag parenteral verabreicht wird.

8. Verwendung nach Anspruch 1, wobei das Medikament die Proliferation der natürlichen und adaptiven regulatorischen T-Zellen induziert.

## Revendications

1. Utilisation du facteur de croissance épidermique (EGF) et du peptide de libération de l'hormone de croissance 6 (GHRP-6) pour la fabrication d'un médicament destiné au traitement et à l'amélioration de troubles affectant le système nerveux central chez un patient souffrant d'un symptôme ou de complications associés à une démyélinisation, à une dégénérescence neuronale et à la mort de cellules neuronales par apoptose ou par nécrose, les maladies mentionnées ci-dessus présentant une étiologie auto-immune.

2. Utilisation selon la revendication 1, dans laquelle l'EGF est l'EGF humain.

3. Utilisation selon la revendication 2, dans laquelle l'EGF humain est obtenu à partir d'une source naturelle, par le biais d'une technologie recombinante ou par synthèse chimique.

4. Utilisation selon l'une quelconque des revendications 1 à 3, dans laquelle l'affection du système nerveux central est :
a. la sclérose en plaques ;
b. la neuromyélite optique.

5. Utilisation selon l'une quelconque des revendications 1 à 4, dans laquelle on administre la combinaison EGF-GHRP-6 par voie intraveineuse, intramusculaire ou intrapéritonéale ou en utilisant un dispositif à libération contrôlée.

6. Utilisation selon l'une quelconque des revendications 1 à 5, dans laquelle on administre le médicament par voie parentérale selon un schéma thérapeutique établi sur une période de 20 à 30 jours, selon un dosage compris dans la plage de 5 à 10 µg de chaque ingrédient considéré indépendamment, par kilogramme de poids corporel du patient par jour.

7. Utilisation selon l'une quelconque des revendications 1 à 6, dans laquelle on administre le médicament par voie parentérale au cours de phases de rémission, sur une période s'étendant jusqu'à 130 jours, selon un dosage compris dans la plage de 1 à 5 µg de chaque ingrédient considéré indépendamment, par kilogramme de poids corporel du patient par jour.

8. Utilisation selon la revendication 1, dans laquelle le médicament induit la prolifération de lymphocytes T régulateurs naturels et adaptatifs.
